Europäisches Patentamt

European Patent Office

Office européen des brevets

(11) Publication number : **0 478 279 A2**

# (12) EUROPEAN PATENT APPLICATION

(21) Application number : **91308696.3**

(22) Date of filing : **24.09.91**

(51) Int. Cl.⁵ : **A61L 27/00**

(30) Priority : **26.09.90 US 588646**

(43) Date of publication of application :
**01.04.92 Bulletin 92/14**

(84) Designated Contracting States :
**BE DE ES FR GB IT NL SE**

(71) Applicant : **Bristol-Myers Squibb Company**
**345 Park Avenue**
**New York, N.Y. 10154 (US)**

(72) Inventor : **Hillegass, Donald V.**
**6300 West Five Mile Road**
**Franksville, Wisconsin (US)**
Inventor : **Woodruff, Eric J.**
**1649 Chatham Street**
**Racine, Wisconsin (US)**

(74) Representative : **Coleman, Stanley et al**
**MATHYS & SQUIRE 10 Fleet Street**
**London EC4Y 1AY (GB)**

(54) **Biocompatible body implant having a textured surface.**

(57)   A soft flexible polymeric biocompatible device is provided for non-cardiovascular reconstruction, augmentation, or indwelling tissue expansion. The polymeric implant (1) has a three dimensional textured polymer membrane surface (7) of controlled porosity and thickness with randomly distributed interconnecting cells (9) having cell sizes of about 90-500 microns.

Fig.1.

EP 0 478 279 A2

## BACKGROUND OF THE INVENTION

### 1. Field of the Invention

This invention relates to non-cardiovascular medical implants particularly suitable for purposes of reconstruction or augmentation in the body. More specifically, the medical implants can be used to ameliorate a traumatically or surgically induced condition, such as a mastectomy, or to correct or remedy defects in the breasts, chin, cheeks, forehead, mastoid, buttocks, or in muscle areas such as the pectoral muscle.

This invention also relates to application as an indwelling tissue expander used in reconstruction following mastectomy, remedial breast surgery, corrective surgery for skin imperfections such as scars and burns, male pattern baldness, and the like.

In a particularly preferred embodiment, the present invention relates to a breast prosthesis having a uniquely textured membrane surface designed to enhance the bio-compatibility of the implant, avoid its rejection by the host body, eliminate or substantially minimize capsular contraction around the prosthesis, and reduce soft tissue/implant interfacial motion.

### 2. Description of the Prior Art

Breast reconstruction following a partial or complete mastectomy has received widespread medical attention and acceptance. Breast augmentation or reconstruction is usually carried out by placing an implant either beneath the pectoralis muscle or subglandular.

U.S. Patent No. 4,643,733 to Becker discloses a reconstruction implant comprising an inflatable flexible prosthesis. The implant, in its deflated state, is surgically placed beneath the breast and expanded to the desired shape by percutaneous injection with a fluid such as saline.

U.S. Patent No. 4,413,359 to Akiyama discloses the use of silicones and fluoropolymers as medical plastics because of their compatibility with tissue and stability in living organisms.

U.S. Patent No. 3,700,380 to Kitrilakis discloses a lining or surface containing a plurality of microcavities or pockets which is compatible with blood or living tissue and forms a tenacious base or anchor for pseudointimal growth and tissue ingrowth without interfering with the normal metabolic process of the cells. The microcavities are formed by providing particles or fibers onto the base material while the material is soft, causing the material to harden, and thereafter removing the particles or fibers leaving microcavities. Microcavities can be formed by employing particles or granules of salt crystals having a selective size and shape distribution and wherein the typical depth of the microcavities is between 0.002 and 0.02 inches (50.8 and 508 microns.)

U.S. Patent No. 4,892,544 to Frisch relates to a method for forming porous surfaced polymeric bodies by adhering water-elutable particles to the outside of a mandrel, and then coating the mandrel with a fluid elastomeric composition, causing the elastomeric composition to harden or set up, then dissolving the particles and removing the elastomer from the mandrel. Sugar and salt in different sizes can be used to control pore size. The elastomeric bodies can be used in making mammary prosthesis, tissue expanders, drug releasing implants, blood storage bags or tubular bodies, such as vascular prostheses.

U.S. Patent No. 4,906,423 to Frisch relates to a method for making a polymeric body having a porous surface, wherein the porous surface is obtained from a leachable foam having open pores. The foaming composition is then contacted with a polymeric body and leached.

U.S. Patent No. 3,376,238 to Gregorian relates to microporous polyolefin films containing a plurality of pores having an average diameter of 0.1 to 10 microns prepared from a mixture of a polyolefin and a pore forming solid.

U.S. Patent Nos. 4,281,669; 4,355,426; 4,374,669; 4,459,252; 4,627,836; all to MacGregor, are divisional patents having a common parent, and relate to cardiovascular implants. The MacGregor patents disclose porous surfaces having a network of interconnected interstitial pores below the surface in fluid flow communication with the surface pores.

U.S. Patent No. 3,992,725 to Homsy relates to a porous, fibrous material suitable for in vivo implantation, preferably formed of carbon or graphite fibers bonded with sintered polytetrafluorethylene to expose the maximum amount of fibrous surface.

U.S. Patent No. 4,604,762 to Robinson relates to an arterial graft prosthesis having a core zone of a porous elastomer, an inner zone of a solid elastomer inside the core zone, and an outer zone of porous elastomer outside the core zone. Formation of the porous core utilizes water elutable particles such as salt or sodium bicarbonate homogeneously mixed with a polyether-polyurethane material to form a slurry that is coated on a mandrel to eventually form a tubular graft which simulates an artery. The pore sizes vary from 5 to 100 microns.

U.S. Patent No. 4,199,864 to Ashman relates to dental implants having a porous surface provided by a salt elution method.

U.S. Patent No. 2,688,139 to Jardon relates to a muscle actuated prosthesis for emplacement in an eye socket.

U.S. Patent No. 3,930,979 to Vallance relates to acid elution of solid particulate additives from a synthetic sheet to prepare porous diaphragms for use in an electrolytic cell.

U.S. Patent No. 3,980,613 to Bachot et al relates to the use of asbestos fibers in water and a fluorinated polymeric resin latex with a pore forming agent.

U.S. Patent No. 4,003,818 to Juillard relates to microporous membranes formed from a homogeneous paste of a pore forming filler substance and a latex intended for use as a diaphragm in an electrolysis cell.

Ersek, "Molecular Impact Surface Textured Implants" (undated paper) discloses that implants generally cause a foreign body reaction and inflammation where the host attempts to either eliminate or encapsulate the prosthesis. With breast implants, capsular contraction around the implant is a principal cause for dissatisfaction and the need for reaugmentation.

Unless the breast implant is biocompatible with the host body and simulates the body tissue around the breast augmentation area, the body tissue tends to encapsulate the implant, contract at this area, squeeze it into a generally spherical shape, and eventually isolate it from the body tissues. In order to reduce the body rejection phenomena and to stabilize the implant, attempts have been made to modify the surface of the prosthesis by using porous implants.

Homsy et al, "Porous Implant Systems for Prosthesis Stabilization," Clinical Orthopaedics and Related Research, vol. 89, pages 220-235 (1972), disclose the reason for using porous materials for the purpose of implantation is to enhance the physiological interaction between the host and the implant. The use of porous implants results in a more effective mechanical integration of the implant with the host. However, porous implants have had limited applications and, so far as is known, are not satisfactory for breast augmentation. This is due to:

1. Limited uses and studies;
2. No matching or pore size to tissue needs;
3. Capsule origin, cause and pathology are still unclear in many respects;
4. Three dimensional textured foam is very new technology. Porous implants are historically two dimensional in design, with peaks and valleys with no undercutting.

Surface textured implants have also been suggested for increased prosthesis stabilization. It is generally believed that the use of surface textured implants allows the growth of host fibrous tissue into and around the interstices of the implant surface thereby producing a mechanical bond between the host and the implant.

Kiraly and Hillegass, "Polyolefin Blood Pump Components," Synthetic Biomedical Polymers, Concepts and Applications, at pages 59-71 (1980), disclose blood pump diaphragms have been compression molded from Hexsyn™ rubber (a high flex polyolefin rubber made by the Goodyear Tire and Rubber Co., Akron, Ohio). The Hexsyn™ diaphragms were coated with aldehyde treated gelatin after the diaphragm outer surface was textured by a salt casting technique to produce a porous textured surface layer approximately 100 microns thick and having a pore size of 10 to 50 microns. The textured surface was established to mechanically hold a biolized coating of aldehyde treated gelatin for blood compatibility.

The term "biolization" refers to aldehyde treated natural tissues and protein coatings which are believed to owe their blood compatibility to the crosslinked protein on the surface.

Kambic et al, "Biolized Surfaces as Chronic Blood Compatible Interfaces", Chapter 8, pages 179-198 in Biocompatible Polymers, Metals, and Composites, edited by M. Szycher, (Technomic Publishing Co., Lancaster, Pennsylvania, 1983) discloses various means that have been used to enhance or increase the level of blood compatibility of polymers, primarily in connection with artificial heart implants.

Murabayashi et al, "Biolized Polyurethane Sponge Vascular Graft For the Study of Compliance Effect", (The 13th Annual Meeting of the Society for Biomaterials, June 2-6, 1987 New York, New York), discloses sponge graft substrates prepared from polyurethane by the salt casting method.

## SUMMARY OF THE INVENTION

In accordance with the present invention a soft flexible polymeric biocompatible implant is provided for purposes of non-cardiovascular reconstruction or augmentation. The polymeric implant has a three dimensional textured surface of controlled porosity and thickness with randomly distributed interconnecting cells having a cell size of about 90 to 500 microns.

The polymeric implants of the present invention are particularly suitable as permanent breast implants since they minimize or substantially eliminate adverse reaction by rejection or capsular formation.

## BRIEF DESCRIPTION OF THE DRAWINGS

Figure 1 is a perspective view of a breast implant made in accordance with the present invention;

Figure 2 is a photomicrograph (100 X enlargement) showing a plan view of a textured surface showing random distribution of interconnecting pores.

Figure 3 is a photomicrograph (100 X enlargement) showing a cross-sectional view of the textured polymeric surface on a base layer of non-porous polymer;

Figure 4 is a diagrammatic representation of a mold assembly used to prepare a textured shell;

Figure 5 is a top view of the mandrel used as a mold;

Figure 6 is a side view of the mandrel used as a mold;

Figure 7 is a diagrammatic representation of the operation of the mold assembly.

## DESCRIPTION OF THE PREFERRED EMBODIMENTS

Although implants have been widely used in various parts of the human body, including implants for the vascular system, the general considerations and criteria for successful implants vary for different body organs based upon physiology function matching and localized tissue reaction and rejection phenomena. For example, the considerations for successful implantation in the vascular system are different from the criteria required for successful implants for breast reconstruction.

For breast implants, it has been theorized that the surface of the polymeric shell portion of the prosthesis should be textured with a three dimensional surface of interconnected cells. The porosity, density and size of these cells are critical if the device is to be successful as a permanent implant which minimizes or substantially eliminates capsular formation and tissue inflammation in the breast area.

In accordance with the present invention, the individual cell sizes in the textured surface of the implant can vary from about 90 to 500 microns. Cell sizes greater than 500 microns can be used, but are not as effective. The size of the textured surface becomes too large, allowing no surface strength and uniform pore density and this defeats the purpose of a textured surface. Cell sizes below 90 microns do not allow for proper infiltration of healthy, living body tissue into the textured surface. This tends to defeat the purpose of the textured surface to function as an anchoring means for the surrounding body tissue to support the prothesis, maximize the healing process, and minimize rejection by the body tissue.

Many prior art methods of producing textured polymeric surfaces are based on first forming a textured mold surface by mechanical, chemical or ion etching techniques. These methods do not produce interconnecting cellular pathways in the polymer. The texture is replicated on the polymer by simply casting an appropriate polymeric material against the etched mold surface and curing the polymeric material. The cured polymer is then removed from the mold with a negative of the etched surface reproduced in the surface of the demolded polymer.

This approach is undesirable because the etched or textured cured polymer is difficult to remove from the mold. The depth of the texture is governed by the depth of the etching in the mold surface, which in turn governs the ease of removal from the mandrel or mold. The tooling costs for preparing etched mold surface are quite high. In addition, the geometry of the texture changes with repeated use of the mold or mandrel because of the progressive wear of the etched surface during the demolding operation over a period of time.

The replication of the textured surface on the polymer has no undercutting. "Undercutting" is a term used to describe a three-dimensional reticulated porous matrix on and into the polymer surface. These types of surfaces are not obtainable by molding or casting over a textured mold because of the limitation of part stripping or removal.

Thus, the improvement in this invention is the production of a three dimensional textured surface for a non-cardiovascular medical implant, in contrast to the two dimensional surfaces of the prior art.

This invention results in a sponge-like textured surface that is physically unique and unlike any that has been produced by mechanical, chemical or ion techniques used to texturize molds. Beyond the unique character of the texture, its physical geometry can be controlled and altered to maximize the beneficial attributes of the prosthetic device to control cell size and density, which will result in reduced tissue ingrowth.

The textured rubber surface can be created by the use of a lost salt casting solution technique as disclosed in the aforementioned article by Kiraly and Hillegass, "Polyolefin Blood Components", which is incorporated by reference herein. According to the method of Kiraly and Hillegass, a sufficient amount of reagent grade sodium chloride (NaCl) is pulverized in an appropriate device, such as a Waring blender, and mechanically graded to obtain the desired salt crystal particle size.

By controlling the salt crystal size through grinding and sieving, the size of the resulting pores on the prosthetic device can be directly controlled. Also the porosity or pore density can be controlled and adjusted to meet any predetermined criteria.

The salt is mixed with an approximately equal amount of a solution comprising about 10% by weight of compounded uncured rubber in a hydrocarbon solvent such as toluene, to form a premix. Mixing of the presized NaCl crystals into the solution is performed by simple mechanical mixing until a uniform dispersion is achieved.

In this invention the weight percentage of compounded uncured rubber to hydrocarbon solvent is governed by the desired viscosity of the solution. The viscosity can range from about 10 to 30, preferably about 15 to 25 seconds in a #3 Zahn cup (ASTM viscosity test apparatus).

The weight percentage of NaCl crystals in the rubber/solvent solution is not only governed by viscosity but also by the desired thickness and porosity of the textured surface layer. In general, using a rubber/solvent solution of a constant viscosity and raising the percent of NaCl crystals results in increased porosity and cross-sectional thickness.

Other solvents that can be used include benzene, cyclohexane, acetone, carbon tetrachloride and 1,1,1-trichloroethane.

The premix is then applied to a rubber substrate, the solvent is evaporated off, and the composite is fully cured. Cure conditions for the final step can vary according to the rubber used. As an example, for silicone, a suitable final cure has been found to be about 2 hours at 121°C (250°F).

The salt is then removed from the cured rubber composite surface by washing or eluting with water. This, also, dissolves the salt remaining in the pores, leaving voids or "cells" corresponding in size to the salt crystals.

Verification that salt is removed from the cured shell can be determined by the following testing methods used to detect the presence of residual salt: scanning electron microscopy of the surface and cross-section of the shell; atomic absorption testing can indicate trace levels of sodium (Na) in shell material; tissue culture testing has proven useful in testing for unacceptable levels of residual NaCl; low energy X-ray (25 KV) is able to show individual crystals in the shell.

These methods were used to establish the amounts of residual NaCl and were supported with shell material 7 day implant, intracutaneous, and acute systemic toxicity testing as well as 45 and 90 day implantation of complete devices. All these tests resulted in acceptable results. Residual sodium as measured by atomic absorption spectrophotometry was less than 10 parts per million.

Washing the NaCl crystals out of the cured shell is critical in the formation of the desired pores or voids. As noted earlier, this washing is done using water, preferably, non-pyrogenic water. Suitable processes can be used to accomplish this NaCl removal and these preferably include agitated water wash, water soak/oven dry cycling, or steam flushing.

Suitable water-elutable particles other than salt can be used to produce the textured rubber surface include sugar, sodium bicarbonate, polyvinylalcohol, cellulose, gelatin, polyethylene oxide and polyvinylpyrrolidone.

The rubber substrate shell can be formed from the same premix rubber/solvent solution that is used to form the textured surface, or from something different. In either case, the rubber/solvent solution is contacted onto the surface of an appropriate mold or mandrel to form the substrate shell of the prosthesis.

This rubber/solvent solution forming the substrate is applied stepwise in single layers of about 0.002 inches until a sufficient thickness is built up. Each individual layer is generally dried for about 15 to 90 minutes at ambient conditions. After air drying and final curing, the initial differentiations between layers disappears. The thickness of the total substrate layer generally varies from about 0.0105 to 0.0115 inches.

The textured membrane thereby formed is integrally bonded to, and homogenous with its rubber substrate. The voids define a randomly distributed continuous lattice network of three dimensional interconnecting cells that forms the textured surface of the rubber, thus creating a foam like surface structure.

The textured surface of the mammary implants of the present invention can be controlled with respect to cell size, cell density and thickness. The cell size can be controlled by the size of the salt crystals used in the premix. The salt concentration in the premix controls the cellular density of the final foam. More salt gives more cells per foam volume; less salt, less cells.

The size of the salt crystals closely approximates the resulting cell size of the textured surface. Since with silicone, for example, maximum shrinkage of about 3% can be expected during cure, controlled grinding, sizing and selective sieving of the salt gives a final crystal geometry replicated in each individual cell of the textured surface. Sodium chloride crystals with particle sizes of about 90 to 500 microns sieve-cut result in corresponding textured cellular openings of approximately 90 to 500 microns.

Within the premix, the cellular density of the textured polymeric surface is determined by the weight ratio of the salt to the rubber/solvent solution. For silicone, it has been found that when the ratio of the salt to rubber-solvent solution varies from about 0.25:1.0 to about 0.70:1.0, respectively, the surface texture is smoother and the pore distribution is more homogeneous than those achieved with higher ratios.

If this ratio is higher than 0.70:1.0, the surface texture of the polymer tends to be uneven, thicker and less manageable. At this high salt to rubber solution ratio, the resulting textured surface has little strength since it comprises practically all porosity and little non-porous elastomer base substrate. Below 0.25:1, texture uniformity is minimal, due to thinning and sporadic texturing only in certain areas along the surface.

The thickness of the textured membrane depends on the number of coating layers of the premix on the substrate. Textured membranes have been produced with overall thicknesses of up to about 0.125 inches. The textured membrane comprises the textured surface and the non-textured, non-porous base substrate layer.

The premix comprising the dispersion of salt in the uncured rubber-solvent solution can be coated in multiple layers in stepwise fashion, and after air drying and curing, the initial differentiation between layers is lost. Homogeneity is attained throughout the entire thickness of textured membrane. Thus, total thickness of the textured layer is simply dependent upon the number of layers involved in the coating process.

Multiple layer construction eliminates the potential for salt separating out in striations which could occur if the entire layer thickness was accomplished in one pour. Multiple layers also result in better control in building up the desired thickness and adjusting the thickness of the textured surface if necessary.

A totally layered matrix, that is, having no substrate, after air drying, cure, and water wash would yield a textured matrix whose thickness is controlled by the number of layers. The thickness of a single layer pouring of premix solution can vary through a wide range.

Typically, with silicone, the thickness for an air dried and cured textured premix layer, can vary from about 0.015 to 0.020 inches, with a preferred thickness of about 0.0175 inches. A suitable range of overall thickness for the total textured layer can vary from about 0.030 inches to about 0.040 inches, preferably about 0.035 inches.

Control of single layer pouring thickness is attained through the manipulation of production variables such as molding temperature, mold rotation speed, rubber/solvent molding solution viscosity, and the ratio of NaCl crystals to the rubber/solvent solution used.

The weight ratio of NaCl to elastomer/solvent dispersion generally varies from about 0.1 to 2.0.

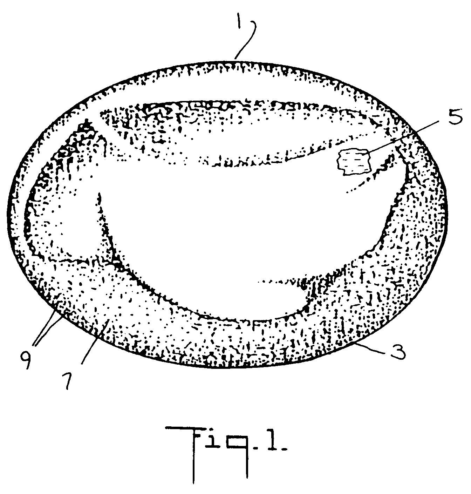

Referring to Figure 1, implant 1 designed to conform to the shape of a breast, comprises a soft flexible expandable outer polymeric shell 3, in this case, silicone rubber, and contains an appropriate fluid 5, such as saline, silicone gel, or other suitable filling medium.

Other polymeric materials that can also be used for the outer shell include but are not limited to elastomers such as polyurethane, ethylene-propylene diene monomer (EPDM), ethylene-propylene rubber (EPR), and polyolefins, such as polyethylene, polypropylene, and ethylene vinyl copolymers such as ethylene-vinyl acetate, ethylene-vinyl chloride, ethylene-vinyl acrylate and the like.

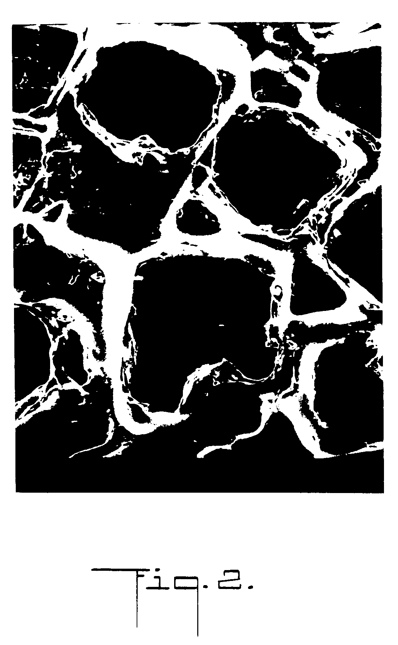

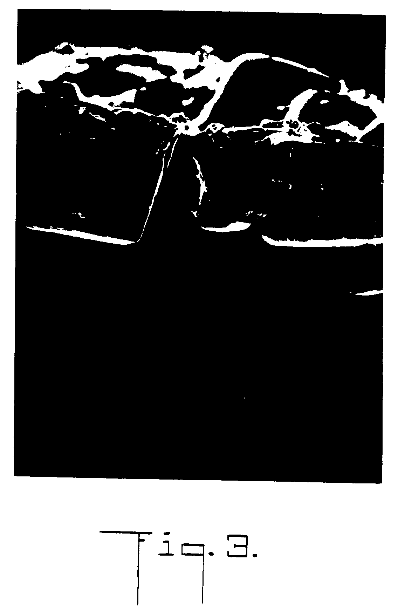

Implant 1 has a textured surface 7 composed of interconnecting cells 9, which are more clearly illustrated in the photomicrograph of Figure 2 showing a top view of the textured surface and Figure 3 showing a cross section with the textured outer surface and the substrate base layer.

Implant 1 that is formed from a silicone rubber shell is a flexible deformable generally hemi-spherically shaped implant for placing subglandular or submuscularly beneath the pectoralis major muscle with breast restoration surgical techniques known to those skilled in the art.

Fabrication of the textured prosthesis begins with the use of a suitably shaped smooth mandrel or mold over which the polymeric substrate will be formed. This substrate comprises a elastomeric base portion of the prosthesis below the textured portion. Its thickness comprises the major portion of the thickness of the prosthesis. Coating of the mold can be accomplished using any one of a number of accepted molding methods including dipping, brushing, spraying or pouring, and the like. The range of molding temperatures can vary from about 20 to 40°C depending on the solvent used.

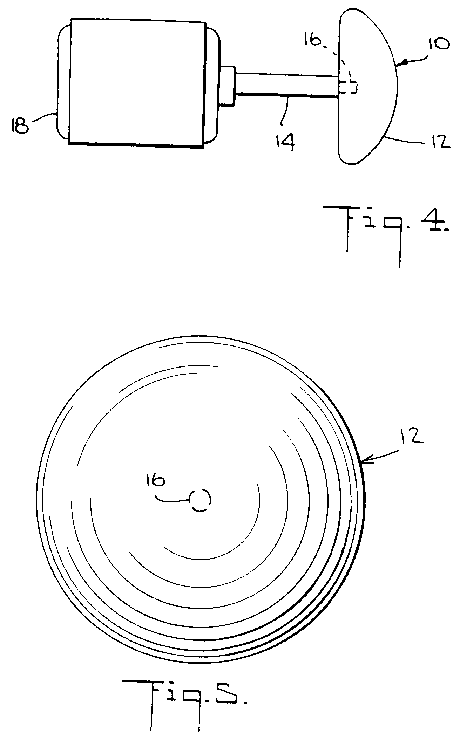

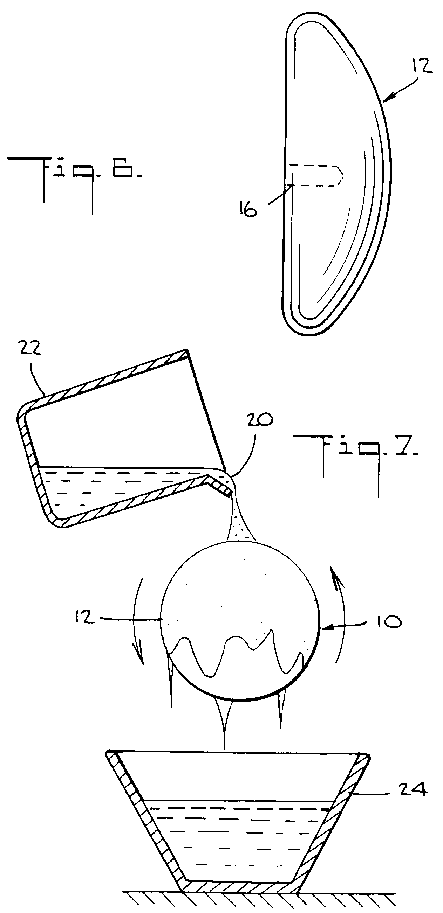

Referring to Figure 4, a mold assembly 10 comprises a mold or mandrel 12 fixedly mounted on a rotating handle 14 through an opening 16 on the back portion of the mandrel 12. Mandrel 12 which is used to form the texturized shell of a breast prosthesis can be made of aluminum. It is shown in greater detail in Figure 5, which is a top view, and Figure 6, which is a side view. The handle 14 is connected to an adjustable motorized rotating unit 18 which rotates the handle 14 and the mandrel 12 attached thereto at predetermined speeds (revolutions per minute). For convenience, the mold assembly 10 is mounted in a horizontal plane.

Referring to Figure 7, which is a diagrammatic representation of the operation of the mold assembly 10, a liquid molding composition 20 in an appropriate container 22 is poured onto mandrel 12 as it rotates. Excess molding composition 20 flows off the mandrel 12 and is captured in vessel 24.

For silicone based on polydimethyl or polymethylvinyl siloxane, or the like, multiple layering results in a non-textured base substrate layer as shown in the lower portion of Fig. 3. This produces a shell of sufficient thickness to fulfill required post cure physical characteristics of about 4-6 pound-force/600-800% elongation for break strength and tear resistance of about 3-5 pound-force.

The specific type of polymeric material used will govern preparation of the base substrate prior to texturing with respect to solvent removal and the extent of cure which is accomplished in a temperature controlled oven for a predetermined amount of time.

For example, silicone materials will generally require a full cure. Ethylene-propylene-diene monomer (EPDM) based polymers will generally undergo a partial cure to provide the base layer with strength, and still allow cure time for reaction with the EPDM based textured layer. Thermoplastic materials do not undergo cure and therefore need only a solvent removal step prior to surface texturing.

After cure or solvent removal, depending upon the specific polymeric material used as the substrate, the polymeric material on the molding apparatus is removed from the oven and allowed to cool to a predetermined temperature.

It is at this point in the manufacturing process that the textured surface is applied. Texture pore size and density is controlled using at least three variables. Taking into account the molding methods used, these variables are: NaCl crystal size, ratio of NaCl to molding elastomer solution, and the number of layers of textured material which are applied.

For a silicone shell, preparation for the texturing operation begins with the sizing of the NaCl crystals. Sizing can be accomplished using standard grinding, sieving, and separation methods.

The resulting sized NaCl crystals are combined with the silicone molding solution which has been formulated with the appropriate siloxane and organic solvent to create a texturizing solution of proper viscosity (about 12-26 seconds in a #3 Zahn cup).

While assuring uniform NaCl suspension, the texturizing solution is applied to the shell mold in layers of about 0.015 to 0.020 inches thickness per layer. All textured layers must be applied to form the total thickness of the textured membrane surface before water washing of the salt crystals. This application is repeated in steps, with sufficient air drying time of about 15-30 minutes between steps to allow for solvent removal, until the desired predetermined cross-sectional thickness of the textured portion is reached.

All solvent must be removed before oven cure. From this point the molded shell/mandrel assembly is placed in a heat controlled oven for a sufficient time to achieve the necessary cure conditions. These conditions are predetermined using ASTM D2084 oscillating disk rheometer guidelines and confirmed with physical property and tissue compatibility testing procedures.

When the cure process is complete, the shell/mandrel assembly is taken from the oven and allowed to cool to ambient temperature. The shell at this time can be left on the mold or removed from the mold by cutting a small circular opening on the back side of the shell and removing the mold through this opening.

Either way, final steps in the preparation of the textured shell include removal of the NaCl crystals to provide the textured foam layer. This can be accomplished with an agitated water wash, a water soak/oven dry cycling system, or a flushing steam clean and with the shell remaining either on or off the mold.

Fabricating a complete prosthetic device next involves molding a seal patch to cover the opening created when the shell is removed from the mandrel.

The seal patch requirement also varies depending on the polymer molding systems used. It does involve the molding of a flat shell, using the same material as the outer shell; including the texturing procedure if desired. From this flat shell, a piece is cut of sufficient size to match and close the open area in the shell that resulted from its detachment from the mandrel. This results in an uncompromised, totally closed shell having a bag-like enclosure.

The shell can then be filled by syringe injection with the desired filling medium such as a saline solution, a silicone gel or other gel system, or an in situ foam, to meet performance requirements as a mammary prosthesis. The injection site is then sealed, for example, with an adhesive. At this point, the prosthetic implant device is packaged and ready for sterilization.

The following examples illustrate but do not limit the scope of the present invention, which is defined by the claims. All parts and percentages are by weight unless otherwise noted.

## Example 1

A textured silicone shell was fabricated by first forming a base substrate with a solution of inhibited chloroplatinic acid catalyzed silicone rubber (10%) in toluene as the casting solution applied in four sequential coats. Each substrate coat had a thickness of about 0.002 inches. The substrate was cast on a mold or mandrel of polished aluminum, with air drying for about 10-30 minutes at 70°F and 50% relative humidity between each coat. The amount of silicone rubber was approximately 10% by weight of the silicone rubber/toluene solution, adjusted to the desired viscosity of about 14 seconds in a No. 3 Zahn cup.

Then, a premix dispersion consisting of a ratio of one part of presized sodium chloride crystals to two parts of the 10 weight percent silicone/ toluene solution was layered onto the aforementioned substrate surface, again in four coating applications with air drying between coats for 10 minutes at 70°F, 50% relative humidity to form a partially cured membrane. The pre-sized sodium chloride crystals consisted of a mixture of equal amounts of salt having particle sizes of 180, 250, 355 and 500 microns. Each premix layer had an average thickness of about 0.007 inches after drying and curing.

The air dried membrane was then exposed to a 50°C vacuum oven for one hour, at 29 inches Hg, then oven cured for 1.5 hours at 150°C. After cure, the membrane was exposed to a thorough dynamic hot tap water wash at a temperature of about 60°C, to remove the sodium chloride. The removal of NaCl was confirmed with a scanning electron microscope evaluation, which showed that no particles of salt were present.

The resulting shell membrane had a three-dimensional outer textured surface of a somewhat rough geometry. The porosity extended through the initially layered base silicone substrate, which was formed from the initial coats of silicone/toluene solution.

## Example 2

The procedure of Example 1 was repeated except that the salt was ground in a mortar and pestle to a very fine consistency of about 10-100 microns and was added at a weight ratio of 1:2 to the catalyzed silicone solution. The resulting surface replicated the crystal size, and the surface texture was much finer compared with Example 1. Its porosity extended through the base substrate due to salt crystal migration from the salt-rubber-solvent dispersion into the base membrane before cure. This resulted in porosity within the membrane after cure and water wash.

Example 3

The method of Examples 1 and 2 demonstrated that placing a salt/silicone dispersion coating directly on an air dried uncured base substrate resulted in salt migration through the base substrate yielding an unwanted porous base substrate in the final product. This example demonstrates an alternate assembly method that eliminated porosity in the base substrate.

The procedure of Example 2 was repeated except that the coated membrane shell was cured, removed from the mandrel, washed to remove the salt, inverted with its textured side in, and returned to the mandrel. Two layers of chlorothane dispersed silicone solution were then applied to the inverted membrane to provide a base membrane seal. The entire assembly was oven cured at 121°C for 1.5 hours. This procedure sealed the porous texturing that had carried through into the base substrate from the top coat salt dispersion step. However, a fine continuous textured surface was retained on the outside of the shell and the overall strength of the membrane was enhanced.

Example 4

The procedure of Example 2 was repeated except that the base substrate was formed from seven layers of the compounded silicone solution followed by a final air drying for one hour at 25°C after the seventh layer was applied. This air dry was followed by a 10 minute oven cure at 121°C, which induced a partial cure to the base substrate. The partial cure was found to be critical in eliminating salt infiltration into the base substrate. Upon cooling, the substrate was then coated with two layers of a premix dispersion of ground salt in a silicone/toluene solution as described in Example 1, wherein the ratio of ground salt to the silicone/solvent solution was 1:2, respectively. The resultant surface was uniformly textured and the substrate remained non-porous. Each premix layer had an average thickness of about .005 to .010 inches after drying and curing. The partial cure was sufficient to assure that no surface damage occurred to the base substrate due to salt migration from the premix dispersion.

Example 5

In Example 4, the partial cure was directly responsible for the elimination of porosity in the base substrate layers. However, the partially cured membrane shell was susceptible to physical damage during the handling between work stations, cure oven, cooling booth, etc. To address these handling concerns, the procedure of Example 4 was repeated. However, rather than inducing a partial cure, the base membrane was fully cured for 1.5 hours at 121°C. The resulting membrane showed uniform texturing with no infiltration of porosity into the base substrate layers.

Example 6

Utilizing the equipment and operation described in FIGS. 4 and 7, the molding of a substrate was performed using a molding composition comprising a solution of inhibited chloroplatinic acid catalyzed silicone rubber in toluene having a viscosity of 14 seconds in a No. 3 Zahn cup, dispensed from a quart jar onto the mandrel rotating at 5 revolutions per minute (rpm).

Approximately 200 to 300 milligrams of the silicone/solvent solution were poured onto the rotating mandrel over a period of about 15 seconds, followed by a 15 minute air dry at ambient conditions. Another 200-300 milligrams of the silicone/solvent solution were poured onto the coated rotating mandrel over a period of about 15 seconds, followed by a 75 minute air dry at ambient conditions. Five additional pours of approximately 200-300 milligrams of the molding composition were each applied to the mandrel in separate steps with 15 minute air drys at ambient conditions between each application.

The last application of the substrate molding composition was followed by a thirty-minute air dry at ambient conditions and then a two-hour oven cure at 121°C (250°F). The substrate-coated mandrel was then removed from the oven and allowed to cool to room temperature. A Teflon⊗ (DuPont Co.) mold release was then applied to the substrate shell coating at the mold interface contacting the handle.

A salt premix dispersion was then prepared using a combination of pre-sized NaCl crystals mixed with the silicone/solvent solution using the same ratio and salt particle size distribution as Example 1. The silicone/solvent solution before mixing was adjusted to a viscosity of 22 seconds in a number 3 Zahn cup. The pre-sized sodium chloride crystals had a uniform particle size distribution that varied from about 180 to 500 microns. Two coats of the salt premix dispersion were then applied to the coated mandrel in amounts of approximately 200 to 300 milligrams with a fifteen-minute ambient dry period between the first and second coats and a final ambient dry of thirty minutes after the second coat, followed by a final cure of two hours at 250°F.

The cured shell was then removed from the mandrel and subjected to a wash cycle of soaking in purified water followed by a flushing rinse with purified water. These wash cycles were repeated three times and followed by an oven dry for fifteen minutes at 250°F.

Although specific embodiments of this invention have been disclosed in the context of a mammary implant, the invention can also be used with implants for reconstruction or augmentation of traumatic or surgically induced defects in the chin, cheeks, forehead, mastoid, buttocks, thigh, and in muscle areas such as the pectoral muscle.

The invention can also be used as an indwelling tissue expander used in reconstruction following mastectomy, remedial breast surgery, corrective surgery for skin imperfections such as scars and burns, male pattern baldness, and the like.

## Claims

1. A method of forming a three dimensional textured membrane surface on a tissue biocompatible prosthesis comprising a hollow outer shell made of a soft flexible polymeric material comprising:

   (a) mixing presized water elutable crystals into a solution comprising a polymeric material in a hydrocarbon solvent;

   (b) contacting said premix to the surface of a hollow non-porous substrate shell to form a coating thereon;

   (c) curing the premix coating at a temperature of about 100°C. to 300°C for about 60 to 120 minutes,

   (d) removing the water elutable crystals from the cured premix coating to thereby form a textured surface layer comprising a random network of three dimensional interconnected cells; wherein the size of each cell varies from about 90 to 500 microns.

2. The method of claim 1 wherein said polymeric material for the premix is selected from the group consisting of polyurethanes, ethylene-propylene diene monomers, ethylene-propylene rubbers, polyolefins, and silicone elastomers.

3. The method of claim 1 wherein the weight ratio of said water elutable salt to said polymer/solvent solution varies from about 0.25:1 to about 0.70:1, respectively.

4. The method of claim 1 wherein said hydrocarbon solvent is selected from the group consisting of toluene, benzene, trichloroethane, acetone, cyclohexane, and carbon tetrachloride.

5. The method of claim 1 wherein the substrate shell comprises a polymeric material selected from the group consisting of polyurethanes, ethylene-propylene diene monomers, ethylene-propylene rubbers, polyolefins, and silicone elastomers.

6. The method of claim 1, wherein the substrate material is silicone rubber and the textured surface is silicone rubber.

7. The method of claim 1, wherein the substrate material is silicone rubber and the textured surface is polyurethane.

8. The method of claim 1, wherein the water elutable crystals are selected from the group consisting of sodium chloride, sugar, sodium bicarbonate, polyvinyl alcohol, cellulose, gelatin, polyethylene oxide and polyvinylpyrrolidone.

9. The method of claim 8, wherein said water elutable crystal is sodium chloride.

10. The method of claim 1, wherein the particle size of the water elutable crystals varies from about 90 to 500 microns.

11. The method of claim 1, wherein the substrate is previously formed in stepwise fashion in single layers of about 0.002 inches.

12. The method of claim 1, wherein the total thickness of the substrate varies from about 0.010 to 0.012 inches.

13. The method of claim 1, wherein the textured surface layer is formed stepwise in single layers of about 0.015 to 0.020 inches.

14. The method of claim 13, wherein the total thickness of the textured surface varies from about 0.030 to 0.040 inches.

15. The method of claim 1, wherein the substrate shell is molded on a mandrel corresponding in shape to the tissue biocompatible prosthesis.

16. A three dimensional textured membrane formed by the method of claim 1.

17. A three dimensional textured membrane surface formed on a tissue biocompatible prosthesis comprising a hollow outer shell made of a soft flexible polymeric material comprising:

   (a) a nonporous substrate shell corresponding in shape to the tissue biocompatible prosthesis, comprising a polymeric material for the premix selected from the group consisting of polyurethanes, ethylene-propylene diene monomers, ethylene-propylene rubbers, polyolefins, and silicone elastomers;

   (b) a textured surface layer coated to said substrate comprising a random network of three dimensional interconnected cells, wherein the size of each cell varies from about 90 to 500 microns, and wherein said textured surface layer comprises a polymeric material selected from the group consisting of polyurethanes, ethylene-propylene diene monomers, ethylene-propylene rubbers, polyolefins, and silicone elastomers.

18. The textured membrane of claim 17, wherein the total thickness of the substrate varies from about 0.010 to 0.012 inches.

19. The textured membrane of claim 17, wherein the total thickness of the textured layer varies from about 0.030 to 0.040 inches.

20. The textured membrane of claim 17, wherein the substrate is silicone rubber and the textured surface layer is silicone rubber.

21. The textured membrane of claim 17, wherein the substrate is silicone rubber and the textured surface layer is polyurethane.

Fig.1.

Fig. 2.

Fig. 3.

Fig. 4.

Fig. 5.

Fig. 6.

Fig. 7.